# EUROPÄISCHE PATENTSCHRIFT

(11) **EP 1 004 326 B1**
(45) Veröffentlichungstag und Bekanntmachung des Hinweises auf die Patenterteilung: **25.06.2003**
(21) Anmeldenummer: 99118975.4
(22) Anmeldetag: 27.09.1999
(51) Int. Cl.: A61M 25/00, A61F 11/00

(54) **Katheter zur Applikation von Medikamenten in Flüssigkeitsräumen des menschlichen Innenohrs**
Inner ear fluid transfer catheter
Catheter de transfert de fluide dans l'oreille interne

(30) Priorität: 19.11.1998 DE 19853299
(43) Veröffentlichungstag der Anmeldung: 31.05.2000
(73) Patentinhaber: Lenarz, Thomas, Prof. Dr. med., 30559 Hannover (DE); Heermann, Ralf, Dr. med., 30559 Hannover (DE)
(72) Erfinder: Lenarz, Thomas, Prof. Dr. med., 30559 Hannover (DE); Heermann, Ralf, Dr. med., 30559 Hannover (DE)
(74) Vertreter: Patentanwälte Thömen & Körner

(56) Entgegenhaltungen:
- WO-A-95/20409
- US-A- 5 195 526
- US-A- 5 476 446
- US-A- 5 713 847

## Beschreibung

Die Erfindung betrifft einen Katheter zur Applikation von Medikamenten in Flüssigkeitsräume des menschlichen Innenohrs nach dem Oberbegriff des Anspruchs 1 oder 6.

Derartige Katheter werden verwendet, um Medikamente in Flüssigkeitsräumen des Innenohrs applizieren zu können. Die Applikation von Medikamenten in das Innenohr kann notwendig werden, um Schädigungen des Hör- und Gleichgewichtsorganes bekämpfen zu können, insbesondere kann es notwendig werden, Symptome wie Hörverlust, Schwindel oder Ohrgeräusche (Tinnitus) behandeln zu können. Unter Tinnitus versteht man die Wahrnehmung eines Schallereignisses, beispielsweise eines Rauschens oder Pfeifens, ohne daß jedoch tatsächlich eine äußere Schalleinwirkung auf das Innenohr einwirkt.

Die Ursache für die Entstehung dieser nur für den Kranken wahrnehmbaren Geräusche sind vielfältig und in ihrer Pathogenese bis heute nicht eindeutig geklärt. Aufgrund der schweren Zugänglichkeit des Innenohres für die Behandlung der jeweiligen Erkrankung sind bisher systemische Applikationen von Medikamenten Standard. Bedingt durch die verursachten Nebenwirkungen ist aber eine effiziente Behandlung der Innenohrerkrankung meist nicht möglich oder nur sehr schwierig durchführbar. Eine Behandlung von Innenohrhörverlusten, Schwindel oder Tinnitus erfolgt heutzutage durch vasoaktive Infusionen, Steroide oder hyperbare Sauerstofftherapie. Anästhetika und Antidepressiva finden neben physikalischen, chirurgischen und psychotherapeutischen Maßnahmen ebenfalls Verwendung. Die systemisch gegebenen Medikamente wirken nicht allein im Bereich des Innenohrs, sondern im gesamten Körper. Dadurch treten sehr oft bereits bei geringer Dosierung Nebenwirkungen auf, die eine gezielte Therapie der Innenohrstörung unmöglich machen. Die Folge davon ist, daß der Mehrheit der Patienten heute nicht effektiv geholfen werden kann. Physikalische, psychotherapeutische und chirurgische Maßnahmen können nur in einem sehr geringen Prozentsatz helfen.

Eine sinnvolle und wirksame Therapie kann lediglich in einer lokalen Gabe von Pharmaka oder der Einspeisung elektrischer Reize in das Innenohr bestehen. Allerdings bereitet bislang die gezielte Applikation von Pharmaka Probleme.

Bei einem bekannten Verfahren werden Medikamente über ein Paukenröhrchen in das Mittelohr mit nachfolgender, unkontrollierter Diffusion in das Innenohr über die Abdeckung des runden Fensters eingespeist. Bei einem derartigen bekannten Verfahren können nur Medikamente appliziert werden, die auch diffusionsfähig sind, was nicht bei allen Medikamenten der Fall ist. Zudem läßt sich die Diffusionsrate auch bei diffusionsfähigen Wirkstoffen kaum sicher bestimmen, vorhersagen und kontrollieren. Daher muß die Medikamentenmenge möglichst groß gewählt werden, damit eine hinreichende Menge der applizierten Substanz per Diffusion in das Innenohr gelangen kann.

Ein gattungsgemäßer bekannter Katheter, welcher von der Firma Neuro-Biometrix USA (jetziger Name: Intra Ear) unter dem Handelsnamen "Round Window µ Cath" und "Round Window E Cath" vertrieben wird, weist an seinem einen Ende eine etwa kugelförmige flexible Endung auf, welche in die Nische der Membran des runden Fensters eingeklemmt wird und dort ihren Halt findet. Diese Endung weist mehrere Öffnungen auf, aus denen das Medikament austreten und durch die Membran des runden Fensters hindurch diffundieren kann. Diese Austrittsöffnungen weisen einen Abstand zur Membran des runden Fensters auf. Auch bei diesem bekannten Katheter können nur voll diffusionsfähige Medikamente eingesetzt werden, da diese Stoffe durch die Membran des runden Fensters hindurch diffundieren müssen.

Gewünscht wird aber eine kontrollierte Direktabgabe von Medikamenten in die Flüssigkeitsräume des Innenohrs, da nur so eine gezielte Therapie der Krankheitssymptome möglich ist. Da nämlich die Lymphe der Flüssigkeitsräume des Innenohrs die zu therapierenden Sinneszellen direkt umspült, eine Blutversorgung für die Sinneszellen direkt aber nicht vorhanden ist, ist die Gabe von Medikamenten über den Blutweg nicht direkt an den Ort der geschädigten Zellen möglich. Daher ist eine kontrollierte Direktabgabe der Medikamente in die Innenohrflüssigkeiten erwünscht, wofür bislang folgende Substanzen und Methoden zur Verfügung stehen:
Steroide und osmotisch wirksame Substanzen, Tinnitus unterdrückende Medikamente aus der Gruppe der membranwirksamen Medikamente und Transmittersubstanzen, Neurotrophine, d.h. von Stoffen, die eine Regeneration oder Protektion der geschädigten Innenohrhörzellen und angegriffenen Hörnervenfasern ermöglichen, Antioxidantien, Gentherapie, Applikation elektrisch geladener Teilchen zur Therapie von Tinnitus und ototoxische Medikamente zur Ausschaltung von Gleichgewichtssinneszellen.

Aus der US-A-5 476 446 ist ein gattungsgemäßer Katheter für die therapeutische Behandlung im Innenohr bekannt, der ein vorzugsweise kugel- oder ovalförmiges Reservoir mit einem inneren Hohlraum zur Aufnahme, Speicherung und Abgabe von Medikamenten umfasst. Das Reservoir weist vorzugsweise mehrere Poren oder eine Öffnung mit einer semipermeablen Membran auf, durch die die Medikamente aus dem Reservoir austreten können, wenn das Reservoir in direkten Kontakt mit dem zu behandelnden Gewebe des Innenohrs gebracht wird. An dem Reservoir kann eine Kapillare mit einer Auslassöffnung angeordnet sein, die in direktem Kontakt mit der Innenohrflüssigkeit oder dem zu behandelnden Gewebe des Innenohrs steht. Außerdem kann an dem Katheter ein leitfähiger Draht zur Messung des elektrischen Potentials des Innenohrgewebes angebracht sein. Vorteilhaft umfasst der Draht an seinem proximalen Ende ein leitfähiges kugelförmiges Element oder ein keulen- oder hakenähnliches Teil, das mit dem Reservoir bzw. der Kapillare verbunden ist und dadurch ebenfalls direkt mit der Innenohrflüssigkeit oder dem Innenohrgewebe in Kontakt kommt. Das Reservoir oder das kugelförmige Element können sich hierbei gegen das Innenohrgewebe abstützen.

Aufgabe der Erfindung ist es, einen Katheter der eingangs genannten Art so auszugestalten, daß er zum einen eine direkte Applikation von Medikamenten in das Innenohr und zum anderen einen sicheren und festen Halt am Innenohr, insbesondere bei der Applikation des Katheters über verschiedene Zugänge, ermöglicht, wobei der Katheter selbst sich nicht negativ auf das Hörvermögen des Patienten auswirken und nach Abschluß der Therapie wieder problemlos entfernbar sein soll.

Diese Aufgabe wird mit einem Katheter nach dem Oberbegriff des Anspruchs 1 gelöst, welcher die Merkmale des kennzeichnenden Teils der Patentansprüche 1, 2 oder 9 aufweist. Außerdem wird diese Aufgabe mit einem Katheter nach dem Oberbegriff des Anspruchs 6 gelöst, welcher die Merkmale des kennzeichnenden Teils des Patentanspruchs 6 aufweist.

Auf überraschend einfache Weise wird so ein Katheter geschaffen, der eine direkte Applikation von Medikamenten in das Innenohr ermöglicht und zum anderen sicher und dauerhaft am Innenohr verankert werden kann. Insbesondere wird mehr Sicherheit bei der Applikation eines Katheters im Innenohr über verschiedene Zugänge erreicht. Hierbei kann der Katheter so ausgestaltet sein, daß er an der Membran des runden Fensters, an der Fußplatte des ovalen Fensters (Steigbügelfuß) oder an der Membran des Saccus endolymphaticus befestigt werden kann, wobei jeweils das Medikament in den Raum hinter der Abdeckung einspeisbar ist. Es ist vorgesehen, daß der erfindungsgemäße Katheter mit einem Medikamenten-Dosiersystem verbindbar ist, insbesondere mit einem Mikrodosiersystem.

Je nach dem, ob der erfindungsgemäße Katheter an der Membran des runden oder an der Fußplatte des ovalen Fensters, an der Membran des Saccus endolymphaticus oder an dem Endost des Knochens des Schneckengehäuses angelegt werden soll, bietet sich als Zugangsweg zu diesen Abdeckungen die posteriore Tympanotomie (vom ausgebohrten Warzenfortsatz aus) als Zugangsweg zu rundem und ovalem Fenster an, wobei aber auch der transmeatale Zugangsweg (durch den Gehörgang) geeignet ist, die erfindungsgemäßen Katheter zu plazieren. Ein kombinierter Zugang kann gegebenenfalls weitere Vorteile bieten. Der Katheter für den Saccus endolymphaticus kann nach Freilegung des Sackes im Bereich des Sinus sigmoideus im Rahmen einer Mastoidektomie (Ausbohren des Warzenfortsatzes) plaziert werden. Der Sack wird hierzu quer im Bereich des oberen und lateralen Anteils inzidiert. Der Katheter wird in die Öffnung entsprechend der Größe des Systems - eingeführt und fixiert sich selbst.

Die Form der Elemente ermöglicht eine suffiziente Abdichtung des eröffneten Flüssigkeitsraumes. Zusätzliche Sicherheit bietet hierbei die Abdeckung mit Bindegewebe. Durch die elastische Aufhängung des Katheters an der jeweiligen Abdeckung ist eine Verschlechterung der Hörschwelle nicht zu erwarten. Ein Entfernen des Katheters ist atraumatisch möglich, ein Verschluß des Lumens kann durch Bindegewebe oder Ähnliches erfolgen. Ein Meßinstrument sowie weitere Applikationsinstrumente erleichtern die Größenauswahl, Positionierung und Fixierung der Katheter. Es ist möglich, verschiedenste Sensoren und Elektroden in die Katheter zu integrieren. Es ist ferner möglich, das Katheterende mit verschiedene Kupplungselementen zu versehen, die eine Anbindung an Mikrodosiersysteme ermöglichen.

Vorteilhaft an dem erfindungsgemäßen Katheter ist ferner, daß er nach Beendigung der Therapie problemlos wieder explantierbar ist. Beim Entfernen des Katheters wird die zurückbleibende Öffnung in der entsprechenden Abdeckung zunächst mit körpereigenem Gewebe abgedeckt und verschlossen, damit keine Flüssigkeit aus dem Innenohr durch die Öffnung nach außen treten kann. Anschließend bildet sich während eines natürlichen Heilungsprozesses Narbengewebe, durch welches die Öffnung endgültig verschlossen wird. Dieses Narbengewebe wirkt sich für den Patienten hinsichtlich seines Hörvermögens nicht nachteilig aus. Bei einem erfindungsgemäßen Katheter gemäß Anspruch 1 oder 2 ist vorgesehen, daß das eine Ende eine quer zu einer Katheterleitung um diese angeordnete Platte zur Anlage an die Membran des runden Fensters (bzw. zur Anlage an das Endost des Knochens des Schneckengehäuses) und eine Kanülenspitze mit einer Austrittsöffnung aufweist, welche durch die Membran hindurchführbar ist, wobei die Platte an ihrem Rand mit wenigstens einem Bügel versehen ist, welcher zur Abstützung gegen den knöchernen Rand des runden Fensters (bzw. des Endosts des Knochens des Schneckengehäuses) ausgebildet ist. Hierbei kann vorgesehen sein, daß die Kanülenspitze mit einem stromleitenden Kabel verbunden ist, daß die Platte einen Durchmesser von 0,7 bis 1,2 mm aufweist und daß die Kanülenspitze aus einem metallischen Werkstoff besteht.

Bei einem derart ausgestalteten erfindungsgemäßen Katheter macht man sich die anatomischen Verhältnisse des runden Fensters und seiner Membran auf überraschend einfache Weise zunutze. Denn bei dieser Ausgestaltung mit Kanülenspitze, Platte und Bügel braucht das eine Ende mit der Kanülenspitze nur durch die Membran des runden Fensters gestochen werden, wonach der oder die Bügel in den knöchernen Rand des runden Fensters einschnappt bzw. einschnappen. Denn dieser knöcherne Rand des runden Fensters weist Vorsprünge auf, die sich zur Anlage von Halteelementen sehr gut eignen.

Bei einem erfindungsgemäßen Katheter gemäß Anspruch 6 ist vorgesehen, daß die Austrittsöffnung an der Stirnfläche der Verdickung angeordnet ist, wobei die Stirnfläche mit wenigstens zwei konzentrisch um die Austrittsöffnung angeordneten Fixationsbügeln aus einem flexiblen Material mit Memoryeffekt versehen ist, wobei die Fixationsbügel durch die Fußplatte des ovalen Fensters hindurchführbar sind und sich gegen die innere Fläche der Fußplatte abstützen. In weiteren Ausgestaltungen dieser Ausführungsform kann vorgesehen sein, daß die Verdickung mit einem Clip versehen ist, welcher sich gegen den Stapes-Schenkel abstützt. Weiter kann vorgesehen sein, daß der Katheter, die Verdickung und die Fixationsbügel von einem entfernbaren Hüllrohr, dessen Innendurchmesser dem Außendurchmesser der Verdickung entspricht, umgeben ist.

Diese Ausgestaltung des erfindungsgemäßen Katheters ist sehr gut dazu geeignet, an der Fußplatte des ovalen Fensters angelegt zu werden. Auch hier geschieht die Anbringung des Katheters an der Fußplatte auf denkbar einfache Weise. Hier wird zunächst in die Fußplatte des ovalen Fensters eine Öffnung eingebracht, wonach der Katheter mit seiner Verdickung so an der Fußplatte des ovalen Fensters angelegt wird, daß die Fixationsbügel durch die Öffnung in der Fußplatte hindurchgeführt werden.

Durch den Memoryeffekt des Materials der Fixationsbügel biegen diese sich nach dem Einführen nach außen, wodurch der Katheter bereits festgelegt ist. Das wahlweise vorgesehene Hüllrohr dient dabei dazu, die Fixationsbügel zunächst in axialer Richtung zu strecken und auf diese Weise die Verdikkung mit den Fixationsbügeln an der Fußplatte des ovalen Fensters anzulegen und die Fixationsbügel durch die Öffnung in der Fußplatte hindurchzuführen. Sobald die Fixationsbügel durch die Fußplatte hindurchgeführt sind und die starre Hülle des Hüllrohrs verlassen, biegen sich die Fixationsbügel durch den Memoryeffekt des Materials bedingt um und stützen sich gegen die Innenfläche der Fußplatte ab. Anschließend kann das Hüllrohr nach hinten herausgezogen und entfernt werden.
Eine Entfernung des Katheters ist durch Zug möglich, wobei das Memory-Metall eine Biegefestigkeit aufweist, welche geringer ist als die Bruchstärke der Fußplatte.

Bei einem erfindungsgemäßen Katheter gemäß Anspruch 9 ist vorgesehen, daß das eine Ende des Katheters mit einem konischen Körper mit vorderer Austrittsöffnung versehen ist, dessen geringster Durchmesser größer ist als der Durchmesser der Katheterleitung, wobei der Körper an seiner Mantelfläche mit wenigstens zwei nach hinten gebogenen hakenförmigen Fixationselementen versehen ist, welche zusammen mit einem vorderen Teilbereich des konischen Körpers durch die Membran des Saccus endolymphaticus hindurchführbar sind und zur Abstützung gegen eine Innenfläche der Membran des Saccus endolymphaticus ausgebildet sind.

Auch mit einem derart ausgestalteten erfindungsgemäßen Katheter ist es sehr einfach, diesen durch die Membran des Saccus endolymphaticus hindurchzuführen und gegen die Membran des Saccus endolymphaticus festzulegen.

Gemäß praktischen Ausgestaltungen der Erfindung bestehen die Kopplungsteile des Katheters aus einem Carbon-Werkstoff oder aus Titandioxid-Keramik. Die übrigen Teile des Katheters können aus Silikon bestehen. Diese Werkstoffe haben sich als besonders geeignet erwiesen, ohne Einflüsse auf das umgebende Gewebe im memschlichen Körper implantiert zu werden.

Weitere Ausgestaltungen und Vorteile werden anhand von bevorzugten Ausführungsbeispielen in der Beschreibung, in der Zeichnung und in den Patentansprüchen näher beschrieben. In der Zeichnung zeigt:
- Fig. 1: ein erstes Ausführungsbeispiel eines erfindungsgemäßen Katheters im Detail von der Seite,
- Fig. 2: ein zweites Ausführungsbeispiel des erfindungsgemäßen Kaheters in perspektivischer Ansicht,
- Fig. 3,4: ein Detail des Katheters aus Fig. 2 von der Seite,
- Fig. 5: eine schematische Ansicht eines dritten Ausführungsbeispiels des erfindungsgemäßen Katheters,
- Fig. 6: eine vergrößerte Darstellung der anatomischen Gegebenheiten eines menschlichen Innenohrs und
- Fig. 7: eine schematische Ansicht eines mit dem Katheter aus den Fig. 2 bis 4 verbundenen Mikrodosiersystems in einer Lage in einem menschlichen Ohr.

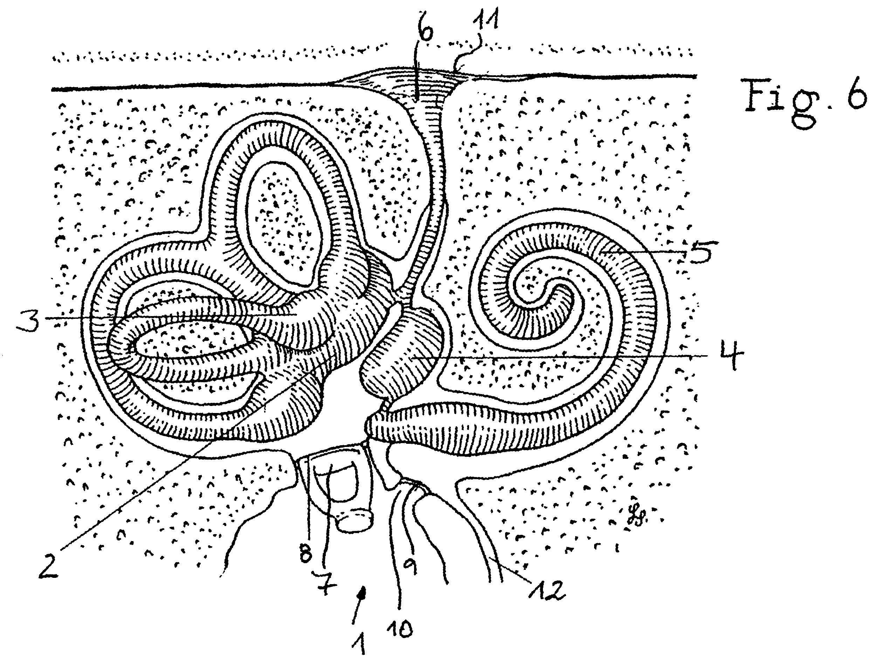

Anhand von Fig. 6 seien zunächst einmal die Verhältnisse in einem menschlichen Innenohr 1 erläutert. Dieses besteht aus dem Utriculus 2, der Ampulle des horizontalen Bogenganges 3, dem Sacculus 4, dem Ductus cochlearis 5 und dem Saccus endolymphaticus 6. Begrenzt wird das Innenohr 1 insbesondere durch die Fußplatte 7 des ovalen Fensters 8, durch die Membran 9 des runden Fensters 10 und durch die Membran 11 des Saccus endolymphaticus 6. Ein weiterer Zugang zum Innenohr 1 stellt der Ductus perilymphaticus 12 dar.

Um Medikamente in das Innenohr 1 applizieren zu können, bieten sich aus physiologischen Gründen insbesondere die Fußplatte 7 des ovalen Fensters 8, sowie die Membran 9 des runden Fensters 10 und die Membran 11 des Saccus endolymphaticus 6 an. Geeignet ist ferner das in Fig. 6 nicht dargestellte Endost (Knochenhaut) des Knochens der Schnecke an, wenn eine Operation ähnlich der Cochlear Implant Operation durchgeführt werden soll.

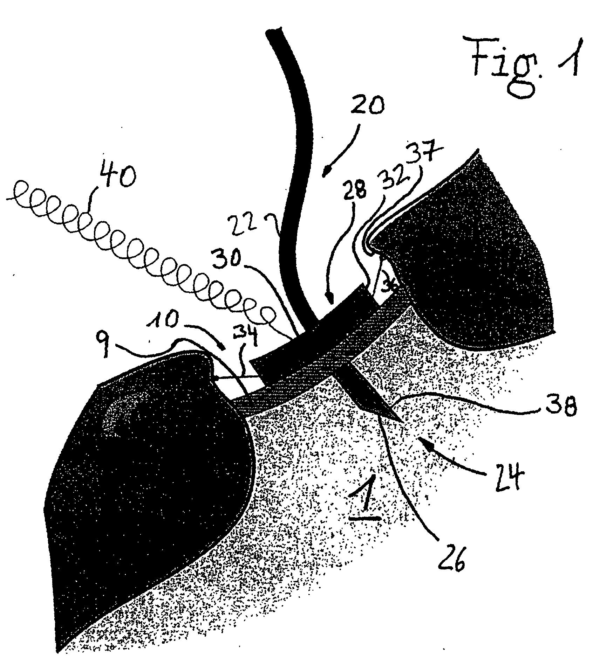

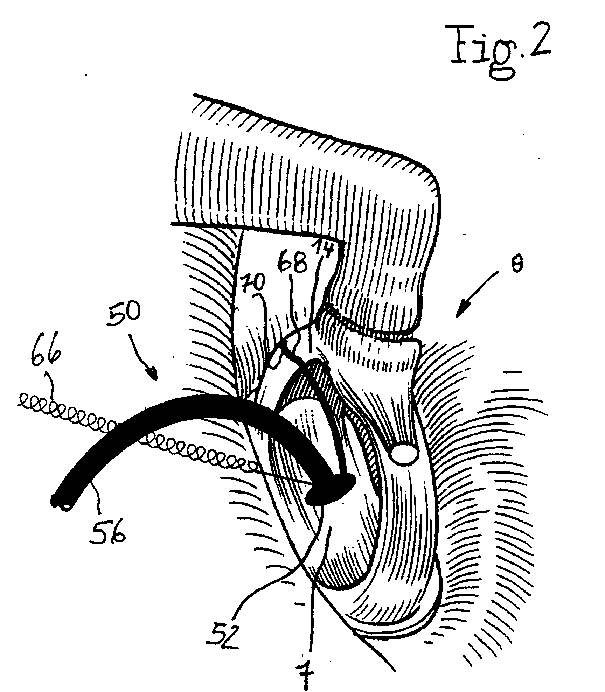

In Fig. 1 ist eine erste Ausführungsform eines erfindungsgemäßen Katheters 20 dargestellt. Dieser Katheter 20 weist eine Katheterleitung 22 auf, von der in Fig. 1 nur ein Abschnitt dargestellt ist. An einem Ende 24 ist der Katheter 20 mit einer Austrittsöffnung 26 versehen. Durch diese Austrittsöffnung 26 kann in die Katheterleitung 22 eingespeistes Medikament aus dem Katheter 20 in das Innenohr 1 austreten.

An dem Ende 24 ist der Katheter 20 ferner mit einem Befestigungselement 28 zur Befestigung des Katheters 20 an der Membran 9 des runden Fensters 10 versehen.

Dieses Befestigungselement 28 besteht aus einer Platte 30, welche quer zur Katheterleitung 22 um diese herum angeordnet ist und sich im Kontakt mit der Membran 9 befindet. Diese Platte 30 ist an ihrem Rand 32 mit zwei Bügeln 34, 36 versehen, welche sich gegen den überhängenden knöchernen Rand 37 des runden Fensters 10 abstützen.

Das eine Ende 24 mit Austrittsöffnung 26 wird durch eine Kanülenspitze 38 gebildet. Diese Kanülenspitze 38 kann aus einem metallischen Material, z.B. aus V4A-Stahl oder aus Titan, bestehen, wobei, wie in Fig. 1 angedeutet, die Kanülenspitze 38 mit einem stromleitenden Kabel 40 verbunden sein kann. Dieses Kabel 40 ermöglicht es, elektrische Reize an das Innenohr 1 zu applizieren oder elektrische Antworten aus dem Innenohr abzuleiten.

Die Platte 30 kann aus Silikon bestehen und kann einen Durchmesser von 0,7; 1,2; 1,7 mm mit einer Dicke von 0,2 mm aufweisen. Die Kanülenspitze 38 kann einen Durchmesser von 0,3 mm und eine Länge von 0,5 mm aufweisen. Die Katheterleitung 22 kann einen Innendurchmesser von 0,3 mm, einen Außendurchmesser von 0,7 mm und eine Länge bis etwa 200 mm aufweisen, wobei die Katheterleitung mit einem noch näher zu beschreibenden Dosiersystem verbunden sein kann. Die sich an den Rand 32 der Platte 30 anschließenden Bügel 34, 36 können eine Länge von 0,7 - 1,0 mm aufweisen.

Der in Fig. 1 dargestellte Katheter 20 läßt sich auch an eine Cochleostomie, d.h. an eine künstlich angelegte Öffnung des Innenohrs anlegen. Hierbei wird der Knochen der Schnecke mit einem Bohrer entfernt und die innere Knochenhaut, das sogenannte Endost, auf ausreichender Fläche freigelegt, etwa in einer Größe von 2 x 2 mm. Bei dem Endost handelt es sich um eine bindegewebige Membran, die als innerste Schicht die Perilymphe umgibt. Nach Freilegen kann sie, ähnlich wie die Membran 9 des runden Fensters 10, punktiert und die Platte 30 mit den Bügeln 34; 36 am umliegenden Knochenrand befestigt werden. Die Platte 30 verhindert auch hier ein zu weites Einführen der Kanülenspitze 38 in das Innenohr 1 und garantiert somit eine definierte maximale Insertionstiefe. Gleichzeitig stellt die Platte 30 ein Abdichtelement in Richtung Innenohr 1 dar. Die maximale garantierte Eindringtiefe vermeidet eine Verletzung der Basilarmembran oder anderer intracochleärer Strukturen. Durch die geringe Einführtiefe wird ein Ausbilden von Fremdkörperreaktionen und/oder einer direkten mechanischen Schädigung vermieden oder doch zumindest stark verringert.

In den Fig. 2 bis 4 ist eine zweite Ausführungsform des erfindungsgemäßen Katheters 50 dargestellt. Dieser Katheter 50 weist eine Verdickung 52 auf, welche ein Ende 54 des Katheters 50 bildet. Auch der Katheter 50 weist eine Katheterleitung 56 auf, von der in den Fig. 2 bis 4 ebenfalls nur ein Teilabschnitt dargestellt ist.

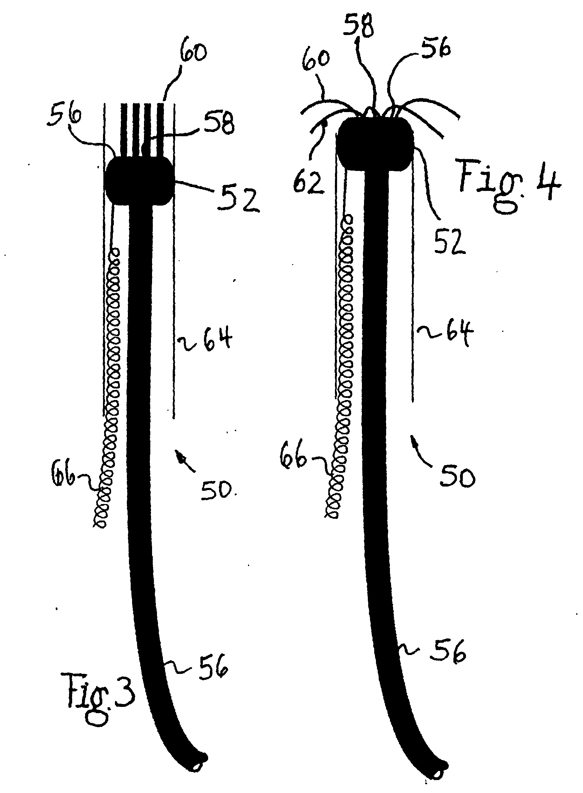

In einer Stirnfläche 56 der Verdickung 52 befindet sich zentral eine Austrittsöffnung 58, durch welche hindurch appliziertes Medikament aus dem Katheter 50 heraustreten kann. Konzentrisch um die Austrittsöffnung 58 der Verdickung 56 herum sind mehrere Befestigungselemente 60 in Form von Fixationsbügeln 62 aus einem Material mit Memoryeffekt angeordnet. Diese sind so ausgestaltet, daß sie sich nach ihrer Streckung wieder in ihre ursprüngliche Lage, wie sie in Fig. 4 dargestellt ist, zurückspreizen.

Um den Katheter 50 in seine in Fig. 2 dargestellte Position an der Fußplatte 7 des ovalen Fensters 8 befestigen zu können, wird die Fußplatte 7 zunächst durchstochen, anschließend werden die Fixationsbügel 62 durch diese Öffnung in der Fußplatte 7 hindurch geführt, wonach sie sich gegen eine Innenseite der Fußplatte 7 festklemmen. Um das Einführen des vorderen Endes 54 des Katheters 50 zu erleichtern, kann ein Hüllrohr 64 verwendet werden, welches in den Fig. 3 und 4 angedeutet ist. In der in Fig. 3 angedeuteten Lage deckt das Hüllrohr 64 die Verdickung 52 und die Fixationsbügel 62 ab, so daß diese in axialer Richtung nach vorn gestreckt werden. Wird in dieser Weise der Katheter 50 auf die Öffnung der Fußplatte 7 aufgesetzt, kann der Katheter 50 nach vorne in Richtung Innenrohr geschoben werden. Sobald die Fixationsbügel 62 durch Öffnung in der Fußplatte 7 hindurch getreten sind und so aus dem Einflußbereich des Hüllrohrs 64 gelangt sind, schnellen sie durch den Memoryeffekt zurück und legen sich an die Innenseite der Fußplatte 7 an. Danach kann das Hüllrohr 64 nach hinten abgezogen werden.

Die Verdickung 52 kann aus Silikon oder aus einem verwandtem Material bestehen und einen Durchmesser von 1 mm und eine Dicke von 0,6 mm aufweisen. Das Innenlumen der Verdickung 52 kann dem Innenlumen der Katheterleitung 52 entsprechen, also etwa 0,4 mm.

Der Katheter 50 kann, ebenfalls wie der Katheter 20 aus Fig. 1, mit einem Kabel 66 versehen sein, welches mit einem Ende direkt in die Stirnfläche 56 der Verdickung 52 endet oder mit einem Fixationsbügel 62 verbunden ist, wenn dieser aus einem metallischen Material besteht.

In dem in Fig. 2 dargestellten Ausführungsbeispiel des Katheters 50 ist die Verdickung 52 mit einem Clip 68, welcher beispielsweise aus Platin besteht, verbunden, welcher sich mit einem Ende 70 gegen einen Stapes-Schenkel 14 abstützt.

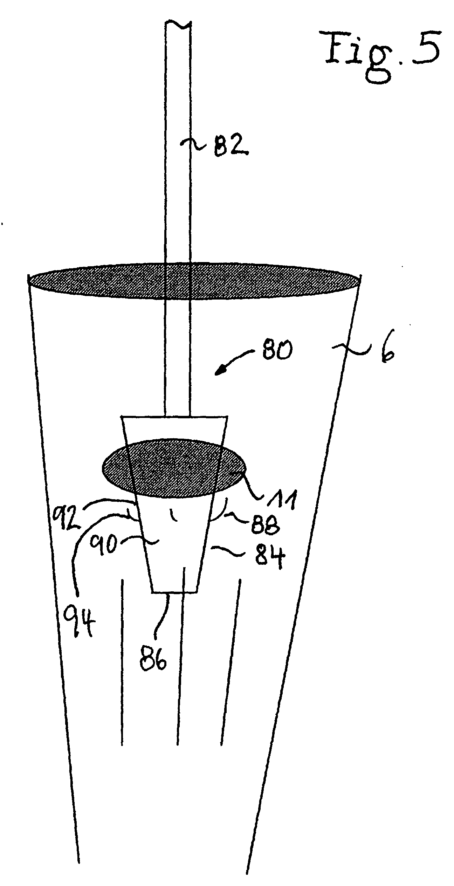

In Fig. 5 ist schematisch ein drittes Ausführungsbeispiel eines erfindungsgemäßen Katheters 80 dargestellt. Dieser Katheter 80 dient dazu, in den Saccus endolymphaticus 6 und durch die Membran 11 des Saccus endolymphaticus 6 hindurch geführt zu werden. Der Katheter 80 weist eine Katheterleitung 82 auf, von der in Fig. 5 nur ein Teilabschnitt dargestellt ist, sowie ein Ende 84 mit Austrittsöffnung 86. Auch dieser Katheter 80 ist mit einem Befestigungselement 88 versehen. Bei dem Katheter 80 gemäß Fig. 5 wird das Ende 84 durch einen konischen Körper 90 gebildet, dessen geringster Durchmesser größer ist als der Durchmesser der Katheterleitung 82. In der Mantelfläche 92 sind als Befestigungselemente 88 dienende hakenförmige Fixationselemente 94 eingebracht. Diese Haken sind mit ihrer Spitze zu der Innenfläche der Membran 11 hin gebogen, so daß nach Einbringung einer Öffnung in die Membran 11 der konische Körper 90 mit den hakenförmigen Fixationselementen 94 zumindest teilweise durch die Öffnung in der Membran 11 hindurch geschoben werden kann, wobei ein Herausrutschen des konischen Körpers 90 aus der Öffnung in der Membran 11 durch die hakenförmigen Fixationselemente 94 verhindert wird. Die Fixationselemente 94 können, ebenfalls wie der Körper 90, aus Silikon bestehen und weisen im gezeigten Ausführungsbeispiel eine Länge von 0,3 mm und einen Durchmesser von 0,1 mm auf.

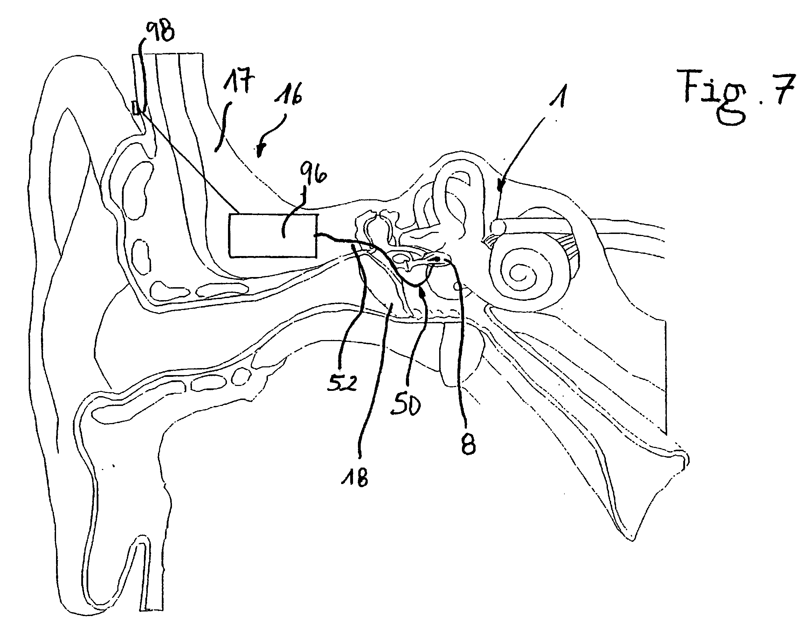

In Fig. 7 ist schematisch ein menschliches Ohr 16 mit Trommelfell 18 dargestellt, welches mit einem Katheter 50 aus den Fig. 2 bis 4 versehen ist. Eine Katheterleitung 52 ist mit einem Mikrodosiersystem 96 verbunden, welches in einem Knochengewebe 17 des Ohres 16 eingepflanzt sein kann. Die Katheterleitung 52 verläuft durch das Mittelohr. Der Katheter 50 ist wie oben zu den Fig. 2 bis 4 mit dem ovalen Fenster 8 verbunden.

Das Mikrodosiersystem 96 kann einen nicht weiter dargestellten Medikamententank, eine Mikropumpe und eine IC-Steuerung aufweisen, welche eine einer Programmierung entsprechende Menge von Medikamenten über die Katheterleitung 52 an das Innenohr 1 freigibt. Das Mikrodosiersystem 96 weist ferner eine an der Körperaußenseite mündende Leitung 98 auf, über welche Medikamente in das Mikrodosiersystem 96 eingespeist werden können. Es ist aber auch möglich, das Mikrodosiersystem so auszugestalten, daß Medikamente mittels einer Spritze in dieses Mikrodosiersystem 96 transkutan eingespeist werden können.

## Patentansprüche

1. Katheter zur Applikation von Medikamenten in Flüssigkeitsräumen des menschlichen Innenohrs durch wenigstens eine Abdeckung (7;9;11) des Innenohrs, wobei der Katheter (20; 50; 80) an einem Ende (24; 54; 84) mit einer Austrittsöffnung (26; 58; 86) für das zu applizierende Medikament, die durch eine Abdeckung (7; 9; 11) des Innenohrs (1) hindurchführbar ist, und mit einem Befestigungselement (28; 60; 88), welches sich direkt gegen die Abdeckung (7; 9; 11) abstützen kann, versehen ist, und wobei ein anderes Ende des Katheters (20; 50; 80) mit einem Medikamenten-Dosiersystem (96) verbindbar ist, **dadurch gekennzeichnet, daß** das Befestigungselement (28) aus einer quer zu einer Katheterleitung (22) um diese angeordneten Platte (30) zur Anlage an die als Abdeckung (9) dienende Membran (9) des runden Fensters (10) des Innenohrs (1), besteht und das eine Ende (24) eine Kanülenspitze (38) mit der Austrittsöffnung (26) aufweist, welche durch die Membran (9) hindurchführbar ist, wobei die Platte (30) an ihrem Rand (32) mit wenigstens einem Bügel (34; 36) versehen ist, welcher zur Abstützung gegen den knöchernen Rand (37) des runden Fensters (10) ausgebildet ist.

2. Katheter nach dem Oberbegriff des Anspruchs 1, **dadurch gekennzeichnet, daß** das Befestigungselement (28) aus einer quer zu einer Katheterleitung (22) um diese angeordneten Platte (30) zur Anlage an das als Abdeckung (9) dienende Endost des Knochens der Schneckenkapsel des Innenohrs (1) besteht und das eine Ende (24) eine Kanülenspitze (38) mit der Austrittsöffnung (26) aufweist, welche durch das Endost hindurchführbar ist, wobei die Platte (30) an ihrem Rand (32) mit wenigstens einem Bügel (34; 36) versehen ist, welcher zur Abstützung gegen einen knöchernen Rand des Endosts ausgebildet ist.

3. Katheter nach Anspruch 1 oder 2, **dadurch gekennzeichnet, daß** die Kanülenspitze (38) mit einem stromleitenden Kabel (40) verbunden ist.

4. Katheter nach einem der Ansprüche 1 bis 3, **dadurch gekennzeichnet, daß** die Platte (30) einen Durchmesser von 0,7 bis 1,2 mm aufweist.

5. Katheter nach einem der Ansprüche 1 bis 4, **dadurch gekennzeichnet, daß** die Kanülenspitze (38) aus einem metallischen Werkstoff besteht.

6. Katheter zur Applikation von Medikamenten in Flüssigkeitsräumen des menschlichen Innenohrs durch wenigstens eine Abdeckung (7;9;11) des Innenohrs, wobei der Katheter (20; 50; 80) an einem Ende (24; 54; 84) eine Verdickung (52) aufweist und mit einer Austrittsöffnung (26; 58; 86) für das zu applizierende Medikament, die durch eine Abdeckung (7; 9; 11) des Innenohrs (1) hindurchführbar ist, und mit einem Befestigungselement (28; 60; 88), welches sich direkt gegen die Abdeckung (7; 9; 11) abstützen kann, versehen ist, und wobei ein anderes Ende des Katheters (20; 50; 80) mit einem Medikamenten-Dosiersystem (96) verbindbar ist, **dadurch gekennzeichnet, daß** die Austrittsöffnung (58) an der Stirnfläche (56) der Verdickung (52) angeordnet ist, wobei die Stirnfläche (56) mit wenigstens zwei konzentrisch um die Austrittsöffnung (58) angeordneten Befestigungselementen (60) in Form von Fixationsbügeln (62) aus einem flexiblen Material mit Memoryeffekt versehen ist, und die Fixationsbügel (62) durch die als Abdeckung (7) dienende Fußplatte (7) des ovalen Fensters (8) hindurchführbar sind und sich gegen die innere Fläche der Fußplatte (7) abstützen können.

7. Katheter nach Anspruch 6, **dadurch gekennzeichnet, daß** die Verdickung (52) mit einem Clip (68) versehen ist, welcher sich gegen den Stapes-Schenkel (14) abstützen kann.

8. Katheter nach Anspruch 6 oder 7, **dadurch gekennzeichnet, daß** der Katheter (50), die Verdickung (52) und die Fixationsbügel (62) von einem entfernbaren Hüllrohr (64), dessen Innendurchmesser dem Außendurchmesser der Verdickung (52) entspricht, umgeben ist.

9. Katheter nach dem Oberbegriff des Anspruchs 1, **dadurch gekennzeichnet, daß** das eine Ende des Katheters (80) mit einem konischen Körper (90) mit der vorderen Austrittsöffnung (86) versehen ist, dessen geringster Durchmesser größer ist als der Durchmesser der Katheterleitung (82), wobei der Körper (90) an seiner Mantelfläche (62) mit Befestigungselementen (88) in Form von wenigstens zwei nach hinten gebogenen hakenförmigen Fixationselementen (94) versehen ist, welche zusammen mit einem vorderen Teilbereich des konischen Körpers (92) durch die als Abdeckung (11) dienende Membran (11) des Saccus endolymphaticus (6) hindurchführbar sind und zur Abstützung gegen eine Innenfläche der Membran (11) des Saccus endolymphaticus (6) ausgebildet sind.

10. Katheter nach einem der Ansprüche 1 bis 9, **dadurch gekennzeichnet, daß** der Katheter (20; 50; 80) aus einem Carbon-Werkstoff besteht.

11. Katheter nach einem der Ansprüche 1 bis 9, **dadurch gekennzeichnet, daß** der Katheter (20; 50; 80) aus Titandioxid-Keramik besteht.

## Claims

1. A catheter for administering medication into fluid cavities of the inner ear in humans through at least one occlusion (7; 9; 11) of the inner ear, the catheter (20; 50; 80) being provided at one end (24; 54; 84) with an outflow aperture (26; 58; 86) for the medication to be administered which is feedable through an occlusion (7; 9; 11) of the inner ear (1), and with an anchoring element (28; 60; 88) which can be supported directly against the occlusion (7; 9; 11), and another end of the catheter (20; 50; 80) being connectable to a medication dosage system (96), **characterised in that** the anchoring element (28) consists of a plate (30) arranged transversely to and around a catheter tube (22) so as to rest against the membrane (9), which serves as the occlusion (9), of the round window (10) of the inner ear (1), and the one end (24) has a cannula point (38) which includes the outflow aperture (26) and is feedable through the membrane (9), the plate (30) being provided at its edge (32) with at least one stay (34, 36) which is configured to anchor against the osseous border (37) of the round window (10).

2. A catheter according to the preamble of Claim 1, **characterised in that** the anchoring element (28) consists of a plate (30) arranged transversely to and around a catheter tube (22) so as to rest against the endosteum of the bone of the cochlear capsule of the inner ear (1), which endosteum serves as the occlusion (9), and the one end (24) has a cannula point (38) which includes the outflow aperture (26) and is feedable through the endosteum, the plate (30) being provided at its edge (32) with at least one stay (34; 36) which is configured to anchor against an osseous border of the endosteum.

3. A catheter according to Claim 1 or 2, **characterised in that** the cannula point (38) is connected to an electroconductive cable (40).

4. A catheter according to any one of claims 1 to 3, **characterised in that** the plate (30) has a diameter of 0.7 to 1.2 mm.

5. A catheter according to any one of claims 1 to 4, **characterised in that** the cannula point (38) is made of a metallic material.

6. A catheter for administering medication into fluid cavities of the inner ear in humans through at least one occlusion (7; 9; 11) of the inner ear, the catheter (20; 50; 80) having at one end (24; 54; 84) a bulbous extension (52) and being provided with an outflow aperture (26; 58; 86) for the medication to be administered, which outflow aperture (26; 58; 86) is feedable through an occlusion (7; 9; 11) of the inner ear (1), and being provided with an anchoring element (28; 60; 88) which can be supported directly against the occlusion (7; 9; 11), and another end of the catheter (20; 50; 80) being connectable to a medication dosage system (96), **characterised in that** the outflow aperture (58) is arranged at the end face (56) of the bulbous extension (52), said end face (56) being provided with at least two anchoring elements (60) in the form of fixing stays (62) arranged concentrically around the outflow aperture (58) and made of a flexible material having a shape memory effect, and the fixing stays (62) being feedable through the footplate (7) of the oval window (8) which serves as the occlusion (7) and being able to anchor themselves against the inner face of the footplate (7).

7. A catheter according to Claim 6, **characterised in that** the bulbous extension (52) is provided with a clip (68) which can anchor itself against the side (14) of the stapes.

8. A catheter according to Claim 6 or 7, **characterised in that** the catheter (50), the bulbous extension (52) and the fixing stays (62) are surrounded by a removable tubular sheath (64) the internal diameter of which corresponds to the external diameter of the bulbous extension (52).

9. A catheter according to the preamble of Claim 1, **characterised in that** the one end of the catheter (80) is provided with a conical body (90) having the front outflow aperture (86), the minimum diameter of which body (90) is greater than the diameter of the catheter tube (82), the body (90) being provided on its outer jacket surface (62) with anchoring elements (88) in the form of at least two backwardly bent hook-like fixing elements (94) which are feedable together with a leading portion of the conical body (92) through the membrane (11) of the endolymphatic sac (6) which serves as the occlusion (11) and are configured to anchor against an inner surface of the membrane (11) of the endolymphatic sac (6).

10. A catheter according to any one of claims 1 to 9, **characterised in that** the catheter (20; 50; 80) is made of a carbon material.

11. A catheter according to any one of claims 1 to 9, **characterised in that** the catheter (20; 50; 80) is made of titanium dioxide ceramic.

## Revendications

1. Cathéter destiné à l'administration de médicaments dans les espaces remplis de liquides de l'oreille interne à travers au moins un recouvrement (7, 9, 11) de l'oreille interne, le cathéter (20, 50, 80) étant pourvu à une extrémité (24, 54, 84) d'une ouverture de sortie (26, 58, 86) pour le médicament à appliquer qui peut être passée à travers un recouvrement (7, 9, 11) de l'oreille interne (1) et d'un élément de fixation (28, 60, 88) qui peut s'appuyer directement contre le recouvrement (7, 9, 11), l'autre extrémité du cathéter (20, 50, 80) pouvant être reliée à un système de dosage de médicaments (96), **caractérisé en ce que** l'élément de fixation (28) est constitué d'une plaque (30) disposée transversalement à un conduit de cathéter (22) et autour de celui-ci et destinée à s'appuyer sur la membrane (9) de la fenêtre ronde (10) de l'oreille interne (1), qui sert de recouvrement (9), et que l'autre extrémité (24) présente une pointe de canule (38) avec l'ouverture de sortie (26), qui peut être passée à travers la membrane (9), la plaque (30) étant pourvue, sur son bord (32) d'une bride (34, 36) conçue pour s'appuyer contre le bord osseux (37) de la fenêtre ronde (10).

2. Cathéter suivant le préambule de la revendication 1, **caractérisé en ce que** l'élément de fixation (28) est une plaque (30) disposée transversalement à un conduit de cathéter (22) et autour de celui-ci et destinée à s'appuyer sur l'endoste de l'os de la capsule de cochlée de l'oreille interne (1) et que l'autre extrémité (24) présente une pointe de canule (38) avec l'ouverture de sortie (26) qui peut être passée à travers l'endoste, la plaque (30) étant pourvue sur son bord (32) d'une bride (34, 36) au moins, conçue pour s'appuyer contre un bord osseux de l'endoste.

3. Cathéter suivant la revendication 1 ou 2, **caractérisé en ce que** la pointe de la canule (38) est reliée à un câble (40) conducteur de courant.

4. Cathéter suivant une des revendications 1 à 3, **caractérisé en ce que** la plaque (30) présente un diamètre de 0,7 à 1,2 mm.

5. Cathéter suivant une des revendications 1 à 4, **caractérisé en ce que** la pointe de la canule (38) est en un matériau métallique.

6. Cathéter destiné à l'administration de médicaments dans des espaces liquides de l'oreille interne humaine à travers un recouvrement au moins (7, 9, 11) de l'oreille interne, le cathéter (20, 50, 80) présentant à une extrémité (24, 54, 84) un épaississement (52) et une ouverture de sortie (26, 58, 86) pour le médicament à administrer, qui peut être passée à travers un recouvrement (7, 9, 11) de l'oreille interne (1), ainsi qu'un élément de fixation (28, 60, 88) qui peut s'appuyer directement contre le recouvrement (7, 9, 11), l'autre extrémité du cathéter (20, 50, 80) pouvant être reliée à un système de dosage de médicaments (96), **caractérisé en ce que** l'ouverture de sortie (58) est disposée à la face (56) de l'épaississement (52), face (56) qui est pourvue au moins de deux éléments de fixation (62) disposés concentriquement autour de l'ouverture de sortie (58), éléments de fixation en forme d'archet (62) en un matériau souple avec mémoire de forme, que les archets de fixation (62) peuvent être passés à travers la plaque d'assise (7) de la fenêtre ovale (8) servant de recouvrement (7) et s'appuyer contre la surface interne de la plaque d'assise (7).

7. Cathéter suivant la revendication 6, **caractérisé en ce que** l'épaississement (52) est pourvu d'une agrafe (68) pouvant s'appuyer contre un branche de l'étrier (14).

8. Cathéter suivant les revendications 6 ou 7, **caractérisé en ce que** le cathéter (50), l'épaississement (52) et les archets de fixation (62) sont entourés d'un tube de gainage (64) amovible, dont le diamètre intérieur correspond au diamètre extérieur de l'épaississement (52).

9. Cathéter suivant le préambule de la revendication 1, **caractérisé en ce qu'**une extrémité du cathéter (80) est pourvue d'un corps conique (90) avec l'ouverture de sortie avant (86), dont le plus petit diamètre est supérieur au diamètre du conduit du cathéter (82), le corps (90) étant pourvu sur sa surface latérale (62) d'éléments de fixation (88, 94) en forme de crochet courbés vers l'arrière, qui peuvent être passés ensemble avec une zone partielle avant du corps conique (92) à travers la membrane. (11) servant de recouvrement (11) du sac endolymphique (6) et qui sont conçus pour s'appuyer contre une surface interne de la membrane (11) du sac endolymphique (6).

10. Cathéter suivant une des revendications 1 à 9, **caractérisé en ce que** le cathéter (20, 50, 80) est en un matériau à base de carbone.

11. Cathéter suivant une des revendications 1 à 9, **caractérisé en ce que** le cathéter (20, 50, 80) est en céramique de dioxyde de titane.
